# EUROPEAN PATENT APPLICATION

(11) **EP 4 647 097 A1**
(43) Date of publication of application: **12.11.2025**
(21) Application number: 24305719.7
(22) Date of filing: 07.05.2024
(51) Int. Cl.: A61M 16/00, A61M 16/04, A61M 16/08

(54) **LIQUID VENTILATION CONNECTOR**

(71) Applicant: Orixha, 69450 Saint-Cyr-au-Mont-d'Or (FR)
(72) Inventor: PERROTTO, Sandrine, 69450 Saint-Cyr-au-Mont-d'Or (FR); LIBARDI, Mickael, 69450 Saint-Cyr-au-Mont-d'Or (FR); PAUBLANT, Fabrice, 69450 Saint-Cyr-au-Mont-d'Or (FR); MARINE, Julien, 49100 Angers (FR); BAJOLET, Aymeric, 54600 Villers-Lès-Nancy (FR); NADEAU, Mathieu, Compton (Québec) J0B 1L0 (CA)
(74) Representative: Icosa

(57) **Abstract**

The present invention relates to an adaptor (10) for connecting a ventilation tube (100) to an endotracheal probe (111) configured to be secured to a patient (P) in need of liquid ventilation, all three elements extending along a revolution axis X. The adaptor comprises a fluidic connection tube (12) extending along the axis X, a locking flange (14) connected to the fluidic connection tube, presenting a main housing (16) for receiving the endotracheal probe, and a main locking element (18) configured to cooperate with the endotracheal probe in order to maintain the endotracheal probe inside the locking flange. When assembled along the X axis, the locking flange exerts a restraining force on the endotracheal probe, said restraining force maintaining the endotracheal probe inside the locking flange.

## Description

### FIELD OF INVENTION

The present invention relates to the field of human liquid ventilation.

### BACKGROUND OF INVENTION

Mechanical ventilators are used to put a critically ill patient under ventilation. More precisely, those ventilators support the ventilatory function of said patients, until the patient's spontaneous (natural) ventilation can be restored. Mechanical ventilators are classically used to move air in and out of the lungs for a limited amount of time.

Beneath the conventional gaseous ventilation strategies, "total liquid ventilation" strategies hold the promise of a radical shift in ventilation. In total liquid ventilation, the patient is ventilated with a breathable liquid having a high solubility for oxygen and carbon dioxide, such as perfluorocarbons, rather than with an oxygen-containing gas mixture. Since the oxygen and air (oxygen, nitrogen and argon) are dissolved in the breathable liquid, the filling of the lungs with the breathable liquid should allow to bring respiratory gas to the lungs, and so to support the respiratory function of the lungs. Since the lungs are completely filled of breathable liquid, the air-liquid interface is removed, which results in an improved lung compliance. Moreover, with a view to other applications than respiratory support, total liquid ventilation allows to use the lungs as heat exchangers with the blood.

Liquid ventilation is based on a repetition of inspirations and expirations, with the ventilation liquid being «renewed» for each inspiration The renewed breathable liquid, enriched in oxygen and brought at the desired temperature, can thus be (re)introduced into the lungs of the patient via an inspiratory pump, an inspiratory tube and endotracheal tube (ETT) (or, as called in the present application "endotracheal probe") and then extracted from the lungs through the endotracheal tube (ETT) (or endotracheal probe), expiratory tube and an expiratory pump. Thus, it is possible to fill the lungs of the subject with the breathable liquid, which exchanges heat and gas with the blood of the subject.

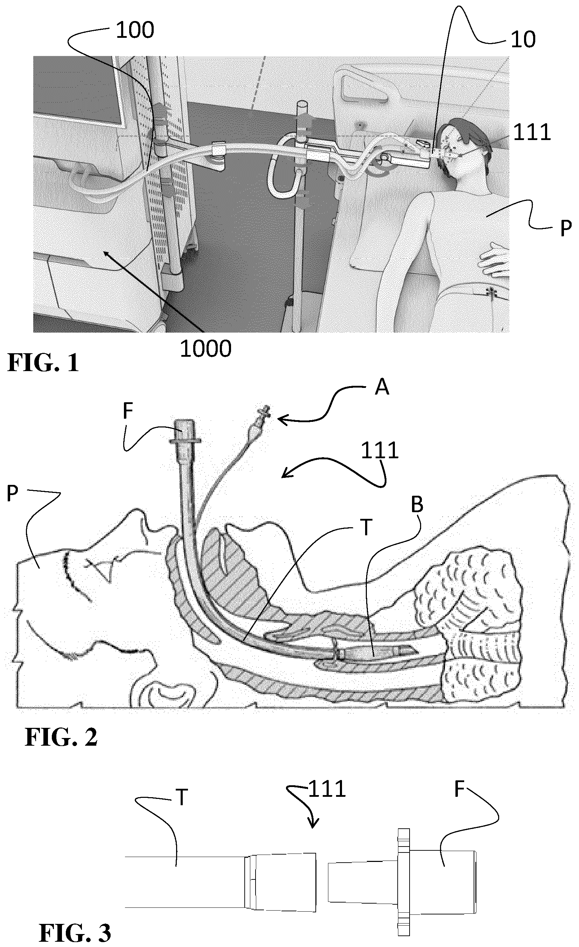

As common part of the management of a patient to be put under total liquid artificial ventilation, the patient has previously been intubated with an endotracheal probe 111 (see figure 2) and ventilated using, most often, a conventional gaseous ventilator, as defined in ISO 80601-2-12:2020 or equivalent. Replacing the patient's endotracheal probe 111 before its connection to the liquid ventilator is clinically not acceptable as it puts the patient at unnecessary risk. Thus, the liquid ventilator must be able to connect to any endotracheal probe 111 already installed on the patient.

However, unlike conventional gaseous ventilation, the pressures involved in the total liquid ventilation are much higher, typically in the range of - 500 to + 1000 mbar compared to atmospheric pressure, or even - 800 to + 2500 mbar within the endotracheal probe. The display of such high positive and negative pressures is explained by the necessary pressure to achieve the desired flow through the probe and the lungs during inspiration or expiration, respectively. Since the flow resistance of a fluid is related to its density, the ventilation with a breathable liquid requires much greater pressures than that needed with the gases used in conventional gaseous ventilation. In the case of conventional gaseous ventilation, pressures would typically be in the -50 to + 120 mbar range. The presence of high negative pressures also induces the risk of air entering the endotracheal probe through the connection locations.

Consequently, the mechanical stresses exerted on the endotracheal probe are much higher than those encountered in gaseous ventilation. In particular, these forces can lead to:
- an involuntary disconnection between the terminal extremity of the ventilation tube of the liquid ventilator and the endotracheal probe,
- an accidental disengagement of the different elements of the endotracheal probe (see detailed description of such a probe further below).

Contrary to conventional gaseous ventilators, a leak regarding liquid ventilators presents far more dangerous consequences, as will be presented further below.

Conventional gaseous ventilators, such as those defined in ISO 80601-2-12:2020, are well known to be equipped with leak detection means. Thus, an accidental disconnection of the gaseous ventilator with the patient sets off an alarm that alerts the practitioner about the leak. Reconnecting has thus little effect on the operation of the gaseous ventilator which, after a maximum of a few seconds or a few breathing cycles, can again provide the needed ventilation as set by the doctor or any other user. Such a disconnection has thus generally little consequence for the patient if detected and solved by the practitioner quickly enough.

On the other hand, a leak in case of a total liquid ventilation would immediately result in the loss of a significant volume of breathable liquid which would quickly lead to the definitive cessation of the procedure, even if the leak was detected quickly and/or automatically by the device (for instance by detecting a drastic drop in pressure during the inspiration of the breathable liquid). The cessation of the procedure is then necessary because either the volume remaining in the liquid ventilator is no longer sufficient to ensure a proper liquid ventilation, or the loss of volume is such that the liquid ventilator is no longer able to accurately determine the volume of breathable liquid in the patient's lungs.

The occurrence of a leak/disconnection during a liquid ventilation procedure is therefore generally irremediable and requires, in an emergency, the shutdown of the procedure. Such a disconnection has thus to be absolutely avoided.

An additional constraint to be taken into account when connecting a liquid ventilator to the endotracheal probe is the mechanical stress on the probe. Although the probe is sometimes secured to the patient by means of a harness which passes, for example, behind the neck, excessive traction on the probe can be a source of leakage or, at the extreme, of involuntary extubation of the patient, with potentially dramatic consequences.

In the context of liquid ventilation, the ventilation tube connecting the liquid ventilator to the patient is complex, incorporating a greater number of sensors and even actuators, and is therefore heavier and bulkier than in conventional gaseous ventilation. The high density of the breathable fluid and the transmission of vibrations through the liquid itself are also likely to exert greater mechanical stress on the probe.

There is therefore a need, in the context of liquid ventilation, for a device and method enabling:
- a quick and safe disconnection of the endotracheal probe intubated inside the patient from the conventional gaseous ventilator to a liquid ventilator, and
- a strong and safe connection between the endotracheal probe intubated inside the patient and the liquid ventilator despite the important pressure and stress exerted on the endotracheal probe by the liquid ventilation.

### SUMMARY

This invention thus relates to an adaptor configured to connect a ventilation tube to an endotracheal probe configured to be secured to a patient in need of liquid ventilation, the ventilation tube, the endotracheal probe and the adaptor all three extending along a revolution axis X, the adaptor comprising:
- a fluidic connection tube extending along the axis X, presenting an upstream extremity configured to be put in fluidic connection with the ventilation tube and a downstream extremity configured to be put in fluidic connection with the endotracheal probe,
- a locking flange connected to the fluidic connection tube, said locking flange presenting a main housing for receiving the endotracheal probe, and a main locking element configured to cooperate with the endotracheal probe in order to maintain the endotracheal probe inside the locking flange,
wherein when assembled along the X axis, the locking flange is configured to exert a restraining force on the endotracheal probe, said restraining force maintaining the endotracheal probe inside the locking flange when liquid is put in circulation between the endotracheal probe and the ventilation tube through the adaptor.

Thus, this solution achieves the above objective. In particular, it allows to obtain a device which enables the connection of the liquid ventilator to the endotracheal probe 111 which can be carried out quickly, typically in less than 30 seconds, ideally in less than 10 seconds, while ensuring a mechanical resistance of the connection to the high liquid pressures for which the endotracheal probe was not designed, in particular:
- the strength and tightness of the connection between adaptor and the endotracheal probe on the one hand, and
- maintaining the fit between the probe tube and the extremity flange of the endotracheal probe.

The device according to the invention may include one or more of the following characteristics, taken in isolation from one another or in combination with one another:
- the main locking element may be displaceable between a resting position and an activated position and is configured, when in its activated position, to cooperate with the main housing in order to maintain the endotracheal probe inside the locking flange, the locking flange being thus configured, when the main locking element is in its activated position, to exert the restraining force on the endotracheal probe,
- the fluidic connection tube may form a first independent block and the locking flange may form a second independent block, the first and second independent blocks being configured to be removably secured to each other,
- the main housing may comprise a secondary locking element presenting a shape configured to interlock with an extremity flange of the endotracheal probe,
- the secondary locking element may comprise at least one groove configured to receive and retain a 3D shaped extremity flange of the endotracheal probe,
- the main housing and the main locking element may be secured to each other with a pivot connection,
- the main housing and the main locking element may be secured to each other with a sliding connection,
- both the main housing and the main locking element may comprise tightening notches enabling a ratchet cooperation between the main housing and the main locking element,
- the adapter may further comprise a sealer configured to be positioned between the downstream extremity of the fluidic connection tube and the securing flange, the sealer being configured to cooperate with the endotracheal probe in order to ensure a tight connection between the fluidic connection tube and the endotracheal probe,
- the sealer may be configured to extend along the axis X, the sealer further presenting a secondary housing configured to receive the downstream extremity of the fluidic connection tube,
- the secondary housing of the sealer and the downstream extremity of the fluidic connection tube may be configured to cooperate by interlocking, remaining free to rotate with regards to each other while cooperating,
- the sealer may comprise at least one internal chamfer configured to maintain a toric joint in abutment contact with the downstream extremity of the fluidic connection tube in order to ensure tightness between the fluidic connection tube and the endotracheal probe,
- the sealer may comprise first longitudinal connection means configured to cooperate with second longitudinal connection means of the locking flange, in order to secure the sealer to the locking flange.

This invention further relates to a connection kit comprising an adaptor according to the here-above listed technical features and an endotracheal probe configured to be secured to a patient in need of liquid ventilation.

This invention also relates to a connection method of a ventilation tube to an endotracheal probe by means of an adapter according to any one of the here-above listed technical features, the method comprising following steps:
- connecting the upstream extremity of the fluidic connection tube to the liquid ventilation tube,
- disconnecting an adapter of the conventional gaseous ventilator tube from the endotracheal probe,
- positioning the endotracheal probe inside the main housing of the locking flange, in such a way that the downstream extremity of the fluidic connection tube is in fluidic connection with the endotracheal probe, and in such a way that the locking flange exerts a restraining force on the endotracheal probe,
- proceeding with the liquid ventilation procedure, which puts the liquid in circulation between the ventilation tube and the endotracheal probe, through the adaptor.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings:
- **Figure 1** is a depiction of a patient connected to a liquid ventilator by means of an adapter according to the present invention,
- **Figure 2** is a schematic depiction of an endotracheal probe intubated inside a patient,
- **Figure 3** is a schematic view of the extremity of an endotracheal probe,
- **Figure 4** is a schematic exploded view of an adapter according to a first embodiment of the present invention,
- **Figure 5** is a perspective view of a main housing of the adapter of figure 4,
- **Figure 6** is a perspective view of a locking flange of the adapter of figure 4,
- **Figure 7** is a perspective view of the mounted adapter of figure 4,
- **Figure 8** is a perspective view of a second embodiment of the adapter of the present invention,
- **Figure 9a** and **9b** are perspective views of a third embodiment of the adapter of the present invention.

### DETAILED DESCRIPTION

### Device

As can be seen on figure 1, the adaptor 10 according to the present invention is configured to connect a ventilation tube 100 of a liquid ventilator 1000 to an endotracheal probe 111 intubated inside a patient P.

The ventilation tube 100 and the tube T of the endotracheal probe 111 are both flexible, allowing a certain amount of flexion and therefore tolerance in terms of the alignment of the patient P with the fluid ventilator.

As can be seen on figure 2, an endotracheal probe 111 is classically configured to be secured to a patient P in need of liquid ventilation. In its simplest form, an endotracheal probe 111, is a tube typically constructed of polyvinyl chloride (PVC) that is placed between the vocal cords through the trachea of a patient P, to provide oxygen and inhaled gases to the lungs (see figure 2). It also serves to protect the lungs of the patient P from contamination, such as gastric contents and blood. As can be seen on figures 2 and 3, an endotracheal probe (or cuffed endotracheal tube) 111 classically comprises:
- a tube T configured to be introduced inside the patients P trachea,
- most endotracheal tubes for use in adults have a tracheal inflatable balloon B (usually called a cuff) near their distal end and are eventually sealed inside the trachea by means of said inflatable balloon B to seal the lower airway at the inflatable balloon B location in the trachea,
- an inflation orifice A to enable the inflation of the balloon B, and
- an extremity flange F presenting a specific 3D shape, configured to be connected to a ventilation tube 100.
As can be seen on figure 3, the tube T and the extremity flange F of the endotracheal probe 111 are removably secured to each other. Therefore, an accidental disengagement of the tube T and the extremity flange F of the endotracheal probe 111 is to be strongly avoided during the ventilation of the patient P. The adaptor 10 according to the present invention presents safety means which avoid such a disengagement, as described further below.

As already mentioned, patients P eligible for liquid ventilation are generally already intubated with an endotracheal probe 111, which provides a seal with the trachea, achieved by inflating an inflatable balloon B. It is then necessary to safely connect said endotracheal probe 111 to a ventilation tube 100 of the liquid ventilator 1000.

The ventilation tube 100, the endotracheal probe 111 and the adaptor 10 all three extend along a longitudinal revolution axis X.

With regard to ventilation, the norm ISO 5361:2016 requires that the extremity flange F shall be a 15 mm male cone and that the extremity of the ventilation tube 100 shall be a 15 mm female cone This norm thus excludes many technical options, *e*.*g*., screwed systems. These conical fittings are not designed to withstand the pressures used in liquid ventilation, hence the necessity to have an adaptor 10 comprising a fitting system compatible with the norms and a locking system ensuring safety.

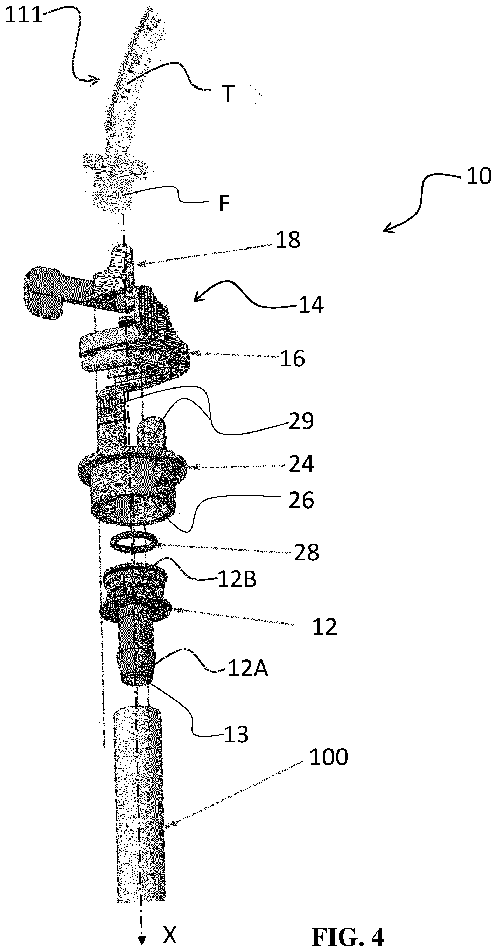

According to the present invention, and as depicted on figure 4, such an adaptor 10 comprises:
- a fluidic connection tube 12 extending along the axis X, presenting an upstream extremity 12A configured to be put in fluidic connection with the ventilation tube 100 and a downstream extremity 12B configured to be put in fluidic connection with the endotracheal probe 111,
- a locking flange 14 connected to the fluidic connection tube 12.

In the present application, the terms "upstream" and "downstream" are defined with regards to the liquid circulating from the liquid ventilator 1000 to the patient P, even if the liquid flow is actually bidirectional.

The different parts of the adapter 10 are preferably injection-moulded parts.

When assembled along the X axis, the locking flange 14 is configured to exert a restraining force on the endotracheal probe 111, more particularly on the junction of the tube T and the extremity flange F of the endotracheal probe 111. The restraining force is exerted in order to maintain, when liquid is put in circulation between the endotracheal probe 111 and the ventilation tube 100 through the adaptor 10:
- firstly, the extremity flange F inside the tube T, and
- secondly the endotracheal probe 111 inside the locking flange 14,

The restraining force is also required before starting the liquid ventilation (meaning before liquid is put in circulation between the endotracheal probe 111 and the ventilation tube 100 through the adaptor 10), and after the end of the liquid ventilation. Maintaining all these elements together allows to guarantee that the assembly is leak-tight before, during and after the liquid ventilation.

The exerted restraining force is preferably a radial force and is typically in the range of 10N to 400N, or even in the range of 10N to 1000N in some embodiments of the locking flange's 14 geometry. Even though the exerted restraining force is radial, it does add and thus increase the friction force between the tube T and the extremity flange F, hence helping in maintaining the extremity flange F inside the tube T. By increasing the restraining radial force, the longitudinal friction force is increased proportionally, as both forces are directly related: the resulting longitudinal force would then typically be in the range of 5N to 200N, or even 5N to 500N in some embodiments.

More precisely, in order to exert said radial restraining force, the locking flange 14 comprises a main housing 16 and a main locking element 18 configured to cooperate together.

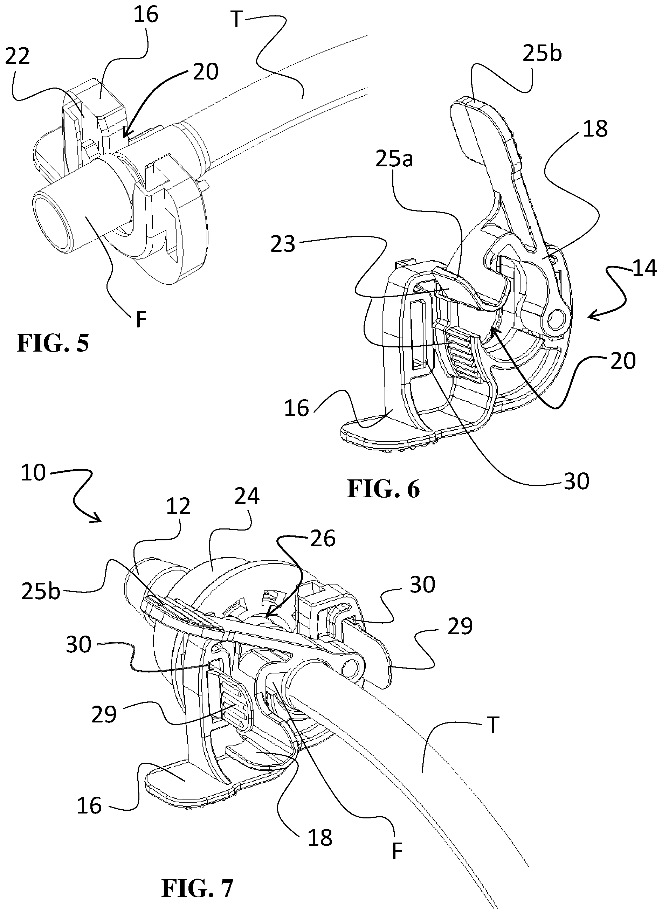

The main housing 16 is configured to receive the endotracheal probe 111. It thus comprises, as can be seen on figure 5, a cavity 20 adapted to receive and preferably retain the extremity flange F of the endotracheal probe 111.

The main locking element 18 (see figures 6, 8 and 9a) is configured to cooperate with the endotracheal probe 111 and the main housing 16 in order to maintain the endotracheal probe 111 inside the locking flange 14.

In some embodiments, in order to introduce the endotracheal probe 111 inside the main housing, the main locking element 18 is displaceable between a resting position and an activated position. The locking flange 14 thus presents a resting configuration in which the locking element 18 is in its resting position and an activated configuration in which the main locking element 18 is in its activated position. The displacement of the main locking element 18 from one position to another can be done by a user or, depending on the embodiment, by an actuator such as a motor. When the main locking element 18 is in its resting position, no force is exerted on the endotracheal probe 111 and the main housing 16 is accessible for a user to position the endotracheal probe 111 inside. On the opposite, the locking flange 14 is configured, when the main locking element 18 is in its activated position, to exert the restraining force on the endotracheal probe 111. More precisely, when in its activated position, the main locking element 18 cooperates with the main housing 16 in order to maintain the endotracheal probe 111 together and inside the locking flange 14. When in its activated position, the main locking element 18 exerts a pressure on the endotracheal probe 111 which is retained by the main housing 16 and thus, the cooperation of the main housing 16 and the main locking element 18 generate a radial force which secures the endotracheal probe 111 together (meaning, by pressing tube T radially against the extremity flange F) and inside the adapter 10.

To sum up, and as can be seen on figures 6, 7, 8 and 9b the main locking element 18 is configured to cooperate with the endotracheal probe 111 in order to maintain:
- firstly, the extremity flange F inside the tube T, and
- secondly the endotracheal probe 111 inside the locking flange 14.

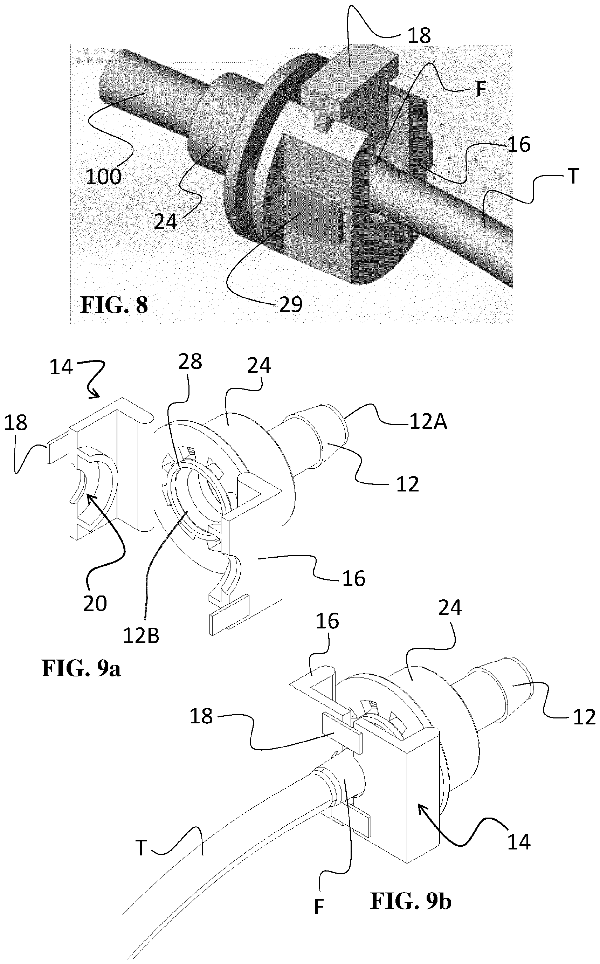

In some embodiments, the main housing 16 and the main locking element 18 are secured to each other with a pivot connection (see figures 6 and 7). In some other embodiments, the main housing 16 and the main locking element 18 are secured to each other with a sliding connection (see figure 8). In that embodiment, the locking flange 14 is preferably fitted with a tab enabling the user to tighten the locking element 18 until enough force (mechanical stress) is exerted on the endotracheal probe 111. Several clamping notches can be present to lock the locking element 18 in position against the main housing 16. Given that endotracheal probes 111 come in different diameters, it is important to offer such latitude to adapt to all probes. Preferably, both the main housing 16 and the main locking element 18 comprise tightening notches 23 enabling a ratchet cooperation between the main housing 16 and the main locking element 18 (see figure 6). Such a ratchet cooperation enables to lock the locking flange 14 in its activated configuration. In some embodiments, the main locking element 18 further comprises a locking tab 25b enabling the user to lock the tightening notches 23. More particularly, the locking tab 25b is an extended arm allowing a proper lever. In some embodiments, an unlocking tab 25a enables the user to unlock the main locking element 18 during uninstallation. This unlocking tab 25a allows the force exerted by the user's finger to be exerted along an axis which facilitates unlocking the tightening notches 23. Preferably, the locking flange 14 can be unlocked with one single action of the user.

In some alternative embodiments (not represented), the locking flange 14 presents a tongue, enabling the locking flange 14 to be tightened and untightened. Other ways of tightening and/or locking the locking flange 14 in its activated configuration are conceivable as long they enable the locking flange 14 to be able to adapt to endotracheal probes 111 of different diameters, which implies having several possible locked configurations. One of those other ways may for example be a screw-type locking mechanism.

In some embodiments, the main housing 16 comprises a secondary locking element 22 presenting a shape configured to interlock with the extremity flange F of the endotracheal probe 111. For example, as can be seen on figure 5, the main housing 16 comprises at least one groove 22 configured to receive and retain the 3D shaped extremity flange F of the endotracheal probe 111. This groove 22 enables to embed the extremity flange F inside the main housing 16, ensuring a stable mechanical connection between the main housing 16 and the endotracheal probe 111. The groove 22 thus cooperates by embedding and/or abutment with the extremity flange F of the endotracheal probe 111. The groove 22 further allows to ensure the correct relative longitudinal positioning of the extremity flange F with regards to the tube T of the endotracheal probe 111 and the ventilation tube 100 of the liquid ventilator 1000. The groove 22 also prevents a user from positioning the flange F upside down inside the main housing 16.

As fluid ventilation involves a periodic movement of breathable fluid towards and from the patient's lungs, a fluid-tight connection between the liquid ventilator 1000 and the patient P is required. The fluidic connection tube 12 presents a channel 13 which ensures the continuity of the fluidic path between the ventilation tube 100 and the endotracheal probe 111. As mentioned above, the downstream extremity 12B of the fluidic connection tube 12 presents a specific shape: a 15 mm male cone fitting the extremity flange F of the endotracheal probe 111.

In order to secure the fitting of the fluidic connection tube 12 to the extremity flange F of the endotracheal probe 111, the fluidic connection tube 12 has to be connected to the locking flange 14. This connection could for example involve a system with latches, spring-loaded pins or equivalent.

More particularly, in the embodiment depicted on figures 4, 7, 8 and 9b, the adapter 10 comprises a sealer 24 configured to fit around the channel 13 of the connection tube 12. The sealer 24 thus presents a secondary housing 26 configured to receive the downstream extremity 12B of the fluidic connection tube 12, so that the sealer 24 is positioned between the downstream extremity 12B of the fluidic connection tube 12 and the locking flange 14. This way, the sealer 24 is also configured to extend along the axis X. The sealer 24 is configured to cooperate with the endotracheal probe 111 in order to ensure a tight connection between the fluidic connection tube 12 and the endotracheal probe 111. To reinforce the tightness, the sealer 24 comprises at least one internal chamfer configured to maintain a toric joint (o-ring) 28 in abutment contact with the downstream extremity 12B of the fluidic connection tube 12 (see figure 4). More precisely, the sealing is ensured by the toric joint 28 on an outer wall of the cone of the extremity flange F of the endotracheal probe 111. Other designs could be envisaged, for example with 2 toric joints (o-ring) instead of one. Another possibility would be, for example, to position the toric joint 28 not around the conic extremity of the endotracheal probe 111, but at its end.

The sealer 24 is configured to be clipped onto the locking flange 14, connecting the fluidic connection tube 12 to the locking flange 14 and thus connecting the ventilation tube 100 of the liquid ventilator 1000 to the patient P. The sealer 24 offers the ability to connect the liquid ventilator 1000 to the endotracheal probe 111 in a limited amount of time. In order to ensure said clipping, the sealer 24 comprises first longitudinal connection means 29 configured to cooperate with second longitudinal connection means 30 of the locking flange 14 (see figures 4, 7 and 8). The first longitudinal connection means 29 can for example be deformable clips. Those deformable clips allow a user to clip/unclip the sealer 24 on the locking flange 14 by simply pinching them between the thumb and forefinger during securing/removal.

The secondary housing 26 of the sealer 24 and the downstream extremity 12B of the fluidic connection tube 12 are configured to cooperate by interlocking, remaining free to rotate with regards to each other while cooperating. The sealer 24 and the connection tube 12 thus fit into each other by means of a pivot connection (see figure 4). This connection allows a rotation along the longitudinal axis X, providing a degree of freedom between the ventilation tube 100 coming from the liquid ventilator 1000 and the endotracheal probe 111. This facilitates the positioning of the adaptor 10 with regards to the endotracheal probe 111.As the ventilation tube 100 is flexible, in combination with the rotation of the fluidic connection tube 12 inside the sealer 24, this allows a certain amount of flexion and therefore tolerance in terms of the alignment of the adaptor 10 with regards to the extremity flange F of the endotracheal probe 111. Such a degree of freedom enables the endotracheal probe 111 to be constantly aligned with the ventilation tube 100 of the liquid ventilator 1000, while limiting the mechanical stresses exerted on the endotracheal probe 111.

Although various means can be implemented to avoid too high pulls on the adaptor 10, any solution for connecting the patient P to the liquid ventilator 1000 through the endotracheal probe 111, the adaptor 10 and the ventilation tube 100 must offer the greatest possible degrees of freedom to enable users to position and secured the adaptor 10 the most appropriately, without hindering access to the patient P (mouth care, etc.) or exerting stress on the endotracheal probe 111.

As previously mentioned, in some embodiments (see figures 4 and 8), the fluidic connection tube 12 forms a first independent block and the locking flange 14 forms a second independent block - hereafter "two independent blocks embodiments". The first and second independent blocks are configured to be removably secured to each other by a user. This securing happens by means of the sealer 24. This embodiment with two independent blocks, the locking flange 14 can be positioned on the endotracheal probe 111 while it is still connected to the conventional gas ventilator without any time constraints. In those embodiments, the sealer 24 is configured to be clipped onto the locking flange 14 when the patient P is being connected to the liquid ventilator 1000.

Another possible design (see figures 9a, 9b) would be to have the locking flange 14 and the fluidic connection tube 12 joined together in a single piece. This embodiment is less optimal because it does not allow the locking flange 14 to be positioned while the endotracheal probe 111 is connected to the gas ventilator.

To sum up, considering the technical problem to be solved by the present invention, the proposed solution addresses the various points as follows:
- enabling a safe and strong the connection between the liquid ventilator 1000 and the endotracheal probe 1 11,
- securing the fit/connection between the tube T and the extremity flange F of the endotracheal probe 111 and the probe tube,
- enabling a rapid installation by the rapid clipping of the different tubes 100, T inside the adapter 10 and the easy tightening of the locking flange 14, and especially regarding the "two independent blocks embodiments" rendering it possible to position the locking flange 14 on the endotracheal probe 111 while the patient P is still connected to the conventional gas ventilator, and
- enabling a safe positioning of the endotracheal probe 111 with regards to the ventilation tube 100 by the absence of mechanical stress on the endotracheal probe 111 by means of at least one degree of freedom.

### Method

The following section describes the sequence of operations that a user, typically a doctor or a nurse, would perform to connect the ventilation tube 100 of a liquid ventilator 1000 to an endotracheal probe 111 configured to be secured to a patient P in need of liquid ventilation.

In the following sequence, it is assumed that the endotracheal probe 111 has previously been connected to a conventional gaseous ventilator as patients P:
(a) the user connects the upstream extremity 12A of the fluidic connection tube 12 to the liquid ventilation tube 100,
(b) the user disconnects the adapter of the conventional gaseous ventilator tube from the endotracheal probe 111,
(c) the user positions the endotracheal probe 111 inside the main housing 16 of the locking flange 14, in such a way that the downstream extremity 12B of the fluidic connection tube 12 is in fluidic connection with the endotracheal probe 111, and in such a way that the locking flange 14 exerts a restraining force on the endotracheal probe 111,
(d) the user proceeds with the liquid ventilation procedure, which puts the liquid in circulation between the ventilation tube 100 and the endotracheal probe 111, through the adaptor 10.

In case the main locking element 18 is displaceable, step (c) includes the user moving the main locking element 18 from its resting position to its activated position in order to put the locking flange 14 in its activated configuration.

At the end of the liquid ventilation procedure, the user may perform the aforementioned operations in reverse order so as disconnect the fluidic connection tube 100 from the endotracheal probe 111 and, optionally, reconnects the endotracheal probe 111 to the tube of a conventional gaseous ventilator:
(a) the user stops the liquid ventilation procedures, which stops the liquid circulation between the liquid ventilator 1000 and the endotracheal probe 111,
(b) in case the main locking element 18 is displaceable, the user moves the main locking element 18 from its activated position to its resting position in order to put the locking flange 14 in its resting configuration,
(c) the user removes the endotracheal probe 111 from the main housing 16 of the locking flange 14,
(d) the user connects the adapter of the conventional gaseous ventilator tube to the endotracheal probe 111,
(e) the user may also disconnect the upstream extremity 12A of the fluidic connection tube 12 from the ventilator tube 100.

In a second embodiment of the method, to be carried out with an adapter 10 presenting two independent blocks, it is assumed the liquid ventilation tube 100 is connected to the liquid ventilator 1000 before use so as to save time for the user:
(a) the user positions the endotracheal probe 111 inside the main housing 16 of the locking flange 14 of the adaptor 10 as per the present invention, in particular, the user positions the 3D extremity flange F of the endotracheal probe 111 into the at least one groove 22 of the main housing 14,
(b) if the main locking element 18 is present, the user moves the main locking element 18 from its resting position to its activated position for example along its pivot connection,
(c) If present, the user locks the tightening notches 23 so that the ratchet cooperation between the main housing 16 and the main locking element 18 exerts a restraining force on the endotracheal probe 111.

Steps (a) (b) and (c) can be performed while the endotracheal probe 111 is still connected to the conventional gaseous ventilator, which reduces the time where the patient is not ventilated.

The method continues as follows:
(d) the user disconnects the tube of the conventional gaseous ventilator from the endotracheal probe 111,
(e) the user rotates the sealer 24 around the rotation axis X so that the first longitudinal connection means 29 aligns with the second longitudinal connection means 30,
(f) the user moves the sealer 24 along the rotation axis X until the first and second longitudinal connection means 29, 30 interlock in order to secure the sealer 24 to the locking flange 14, ensuring a tight connection between the fluidic connection tube 12 and the endotracheal probe 111,
(g) the user proceeds with the liquid ventilation procedure, which puts the liquid in circulation between the ventilation tube 100 and the endotracheal probe 111, through the adaptor.

At the end of the liquid ventilation procedure, the user may perform the aforementioned operations in reverse order so as disconnect the ventilation tube 100 from the endotracheal probe 111 and, optionally, reconnects the endotracheal probe 111 to the ventilation tube of a conventional gaseous ventilator.

## Claims

1. Adaptor (10) configured to connect a ventilation tube (100) to an endotracheal probe (111) configured to be secured to a patient (P) in need of liquid ventilation, the ventilation tube (100), the endotracheal probe (111) and the adaptor (10) all three extending along a revolution axis X, the adaptor (10) comprising:
- a fluidic connection tube (12) extending along the axis X, presenting an upstream extremity (12A) configured to be put in fluidic connection with the ventilation tube (100) and a downstream extremity (12B) configured to be put in fluidic connection with the endotracheal probe (111),
- a locking flange (14) connected to the fluidic connection tube (12), said locking flange (14) presenting a main housing (16) for receiving the endotracheal probe (111), and a main locking element (18) configured to cooperate with the endotracheal probe (111) in order to maintain the endotracheal probe (111) inside the locking flange (14),
**wherein** when assembled along the X axis, the locking flange (14) is configured to exert a restraining force on the endotracheal probe (111), said restraining force maintaining the endotracheal probe (111) inside the locking flange (14) when liquid is put in circulation between the endotracheal probe (111) and the ventilation tube (100) through the adaptor (10).

2. Adaptor (10) according to the preceding claim, wherein the main locking element (18) is displaceable between a resting position and an activated position and is configured, when in its activated position, to cooperate with the main housing (16) in order to maintain the endotracheal probe (111) inside the locking flange (14), the locking flange (14) being thus configured, when the main locking element (18) is in its activated position, to exert the restraining force on the endotracheal probe (111).

3. Adaptor (10) according to any one of the preceding claims, **wherein** the fluidic connection tube (12) forms a first independent block and the locking flange (14) forms a second independent block, the first and second independent blocks being configured to be removably secured to each other.

4. Adaptor (10) according to any one of the preceding claims, **wherein** the main housing (16) comprises a secondary locking element (22) presenting a shape configured to interlock with an extremity flange (F) of the endotracheal probe (111).

5. Adaptor (10) according to the preceding claim, **wherein** the secondary locking element (22) comprises at least one groove configured to receive and retain a 3D shaped extremity flange (F) of the endotracheal probe (111).

6. Adaptor (10) according to any one of claims **2** to **5, wherein** the main housing (16) and the main locking element (18) are secured to each other with a pivot connection.

7. Adaptor (10) according to any one of claims **2** to **6, wherein** the main housing (16) and the main locking element (18) are secured to each other with a sliding connection.

8. Adaptor (10) according to any one of claims **2** to **7, wherein** both the main housing (16) and the main locking element (18) comprise tightening notches (23) enabling a ratchet cooperation between the main housing (16) and the main locking element (18).

9. Adaptor (10) according to any one of the preceding claims, **wherein** the adapter (10) further comprises a sealer (24) configured to be positioned between the downstream extremity (12B) of the fluidic connection tube (12) and the securing flange (14), the sealer (24) being configured to cooperate with the endotracheal probe (111) in order to ensure a tight connection between the fluidic connection tube (12) and the endotracheal probe (111).

10. Adaptor (10) according to the preceding claim, **wherein** the sealer (24) is configured to extend along the axis X, the sealer (24) further presenting a secondary housing (20) configured to receive the downstream extremity (12B) of the fluidic connection tube (12).

11. Adaptor (10) according to the preceding claim, **wherein** the secondary housing (20) of the sealer (24) and the downstream extremity (12B) of the fluidic connection tube (12) are configured to cooperate by interlocking, remaining free to rotate with regards to each other while cooperating.

12. Adaptor (10) according to any one of claims **9** to **11, wherein** the sealer (24) comprises at least one internal chamfer configured to maintain a toric joint (28) in abutment contact with the downstream extremity (12B) of the fluidic connection tube (12) in order to ensure tightness between the fluidic connection tube (12) and the endotracheal probe (111).

13. Adaptor (10) according to claims **9** to **12, wherein** the sealer (24) comprises first longitudinal connection means (29) configured to cooperate with second longitudinal connection means (30) of the locking flange (14), in order to secure the sealer (24) to the locking flange (14).

14. Connection kit comprising an adaptor (10) according to anyone of the preceding claims and an endotracheal probe (111) configured to be secured to a patient (P) in need of liquid ventilation.

15. Connection method of a ventilation tube (100) to an endotracheal probe (111) by means of an adapter (10) according to any one of the preceding claims, the method comprising following steps:
(a) connecting the upstream extremity (12A) of the fluidic connection tube (12) to the liquid ventilation tube (100),
(b) disconnecting an adapter of the conventional gaseous ventilator tube from the endotracheal probe (111),
(c) positioning the endotracheal probe (111) inside the main housing (16) of the locking flange (14), in such a way that the downstream extremity (12B) of the fluidic connection tube (12) is in fluidic connection with the endotracheal probe (111), and in such a way that the locking flange 14 exerts a restraining force on the endotracheal probe (111),
(d) proceeding with the liquid ventilation procedure, which puts the liquid in circulation between the ventilation tube (100) and the endotracheal probe (111), through the adaptor (10).
